(19) Europäisches Patentamt
European Patent Office
Office européen des brevets

(11) EP 2 517 687 B1

(12) EUROPEAN PATENT SPECIFICATION

(45) Date of publication and mention of the grant of the patent:
**12.08.2015 Bulletin 2015/33**

(21) Application number: **11165158.4**

(22) Date of filing: **06.05.2011**

(51) Int Cl.:
***A61K 8/46*** *(2006.01)*
***A61K 8/891*** *(2006.01)*
***A61K 8/894*** *(2006.01)*
***A61Q 9/04*** *(2006.01)*
***A61Q 17/00*** *(2006.01)*
***A61K 8/06*** *(2006.01)*

(54) **Method and kit for depilation**

Verfahren und Kit zur Depilation

Procédé et kit d'épilation

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2011 US 479881 P**

(43) Date of publication of application:
**31.10.2012 Bulletin 2012/44**

(73) Proprietor: **The Procter & Gamble Company**
**Cincinnati, OH 45202 (US)**

(72) Inventors:
- **Smith, Charles, Robert**
  **Wiltshire, SN1 3PS (GB)**
- **Simpson, Naomi, Mary**
  **Twickenham, Middlesex TW2 5QB (GB)**
- **Hewlins, Stuart, Andrew**
  **Woking, Surrey GU24 9LW (GB)**

(74) Representative: **Kohol, Sonia**
**Technical Centres Limited**
**Procter & Gamble Patent Department**
**Rusham Park**
**Whitehall Lane**
**Egham, Surrey TW20 9NW (GB)**

(56) References cited:
**WO-A1-2006/021782**
**GB-A- 2 327 190**
**US-A1- 2004 219 118**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 517 687 B1

Printed by Jouve, 75001 PARIS (FR)

## Description

### FIELD OF THE INVENTION

The present invention relates to a method and kit for depilation.

### BACKGROUND OF THE INVENTION

Depilatory compositions are cosmetic hair removal formulations. They comprise keratin reducing agents, which attack the disulphide bonds in hair to weaken it, such that subsequent gentle scraping and/or wiping completes severance of the hair from the skin and effects hair removal. Commercially, the most common keratin reducing agents are thioglycolates, which are typically formulated at high pH. An unwanted side effect of chemical depilation is that the depilatory composition comes into contact with and must have a relatively long residence time on skin to achieve effective hair removal and this long residence time combined with the alkaline conditions needed for effective hair removal may give rise to skin irritation.

The above problem has been recognized in the art. Reference is made to US 4,401,663 and US 4,424,205 the disclosures of which are similar to one another. These documents teach to use certain compounds as topical analgesics and an example given of a situation in which it is suggested to use such materials is to reduce depilatory irritation. The carrier formulas disclosed to be suitable for formulation with the analgesics include lotions and creams.

Reference is also made to US 2004/0219118, which discloses treatment with a "lipophilic" material before application of a thioglycolate-based reactive depilatory compostion. Lipophilic materials exemplified in this patent application are oils, such as mineral oil. As shown hereinbelow, the present applicants have tested a range of lipophilic materials to determine their ability to prevent thioglycolate penetration and, thereby, their ability to reduce or prevent skin irritation Applicants have surprisingly found that oils, such as mineral oil, have no or a low ability to prevent thioglycolate penetration to the skin. There thus exists a need to develop a pre-treatment composition which better reduces skin irritation.

### SUMMARY OF THE INVENTION

According to a first aspect of the invention, a method of removing hair from skin according to claim 1 is provided.

According to a second aspect of the invention, a depilatory kit according to claim 12 is provided.

### BRIEF DESCRIPTION OF THE DRAWINGS

Fig.1. is a schematic view of a Franz Cell apparatus.

### DETAILED DESCRIPTION OF THE INVENTION

The protective composition used in the method and comprised within the kit according to the invention comprises an emulsion having a hydrophobic continuous phase and a hydrophilic dispersed phase. As demonstrated by the Franz Cell test data, below, the applicants have established that such emulsions are significantly better at preventing the penetration of keratin reducing agent, such as thioglycolate, to the skin, than oil on its own and therefore result in reduced skin irritation. Applicants have established that the use of the present protective composition still permits hair weakening and destruction by the keratin reducing agent, so that reduced skin irritation can be achieved while removing hair. A reduction of thioglycolic acid penetration of 45% or more according to the Franz Cell test method may be shown to correlate to a significant and user-noticeable reduction in irritation.

The protective composition used in the method and comprised within the kit according to the invention comprises an emulsion having a hydrophobic continuous phase. Advantageously, the protective composition comprises 10% to 95%, preferably, 15% to 95%, more preferably 25% to 75% and more preferably still 35% to 65% hydrophobic continuous phase by weight of the protective composition.

The hydrophobic continuous phase may comprise one or more oils. As used herein, the term "oil" includes, but is not limited to any non-aqueous substance that is practically insoluble in water according to the United States' Pharmacopeia (USP) definition in 31/NF 26 Vol. 2 General Notices, Page Xvii. (which, according to that definition, means that more than 10,000 parts of water are needed to dissolve 1 part solute) and is liquid at 20°C.

The oil may be selected from natural oil, synthetic oil, silicone oil and mixtures thereof.

Non-limiting examples of suitable natural oils include Acetylated Castor Oil, Acetylated Hydrogenated Castor Oil, Actinidia Chinensis (Kiwi), Seed Oil, Adansonia Digitata Oil, Aleurites Moluccana Seed Oil, Anacardium Occidentale (Cashew) Seed Oil, Arachis Hypogaea (Peanut) Oil, Arctium Lappa Seed Oil, Argania Spinosa Kernel Oil, Argemone

Mexicana Oil, Avena Sativa (Oat) Kernel Oil, Bertholletia Excelsa Seed Oil, Borago Officinalis Seed Oil, Brassica Campestris (Rapeseed) Seed Oil, Calophyllum Tacamahaca Seed Oil, Camellia Japonica Seed Oil, Camellia Kissi Seed Oil, Camellia Oleifera Seed Oil, Canola Oil, Caprylic/Capric/Lauric Triglyceride, Caprylic/Capric/Linoleic Triglyceride, Caprylic/Capric/Myristic/Stearic Triglyceride, Caprylic/Capric/Stearic Triglyceride, Caprylic/Capric Triglyceride, Carthamus Tinctorius (Hybrid Safflower) Seed Oil, Carthamus Tinctorius (Safflower) Seed Oil, Carum Carvi (Caraway) Seed Oil, Carya Illinoensis (Pecan) Seed Oil, Castor Oil Benzoate, Chenopodium Quinoa Seed Oil, Cibotium Barometz Oil, Citrullus Vulgaris (Watermelon) Seed Oil, Cocos Nucifera (Coconut) Oil, Cod Liver Oil, Coffea Arabica (Coffee) Seed Oil, Coix Lacryma-Jobi (Job's Tears) Seed Oil, Corylus Americana (Hazel) Seed Oil, Corylus Avellana (Hazel) Seed Oil, Cucumis Sativus (Cucumber) Oil, Cucurbita Pepo (Pumpkin) Seed Oil, Daucus Carota Sativa (Carrot) Seed Oil, Elaeis Guineensis (Palm) Kernel Oil, Elaeis Guineensis (Palm) Oil, Gossypium (Cotton) Seed Oil, Helianthus Annuus (Hybrid Sunflower) Oil, Helianthus Annuus (Sunflower) Seed Oil, Hippophae Rhamnoides Oil, Human Placental Lipids, Hydrogenated Canola Oil, Hydrogenated Castor Oil, Hydrogenated Castor Oil Laurate, Hydrogenated Castor Oil Triisostearate, Hydrogenated Coconut Oil, Hydrogenated Cottonseed Oil, Hydrogenated C12-18 Triglycerides, Hydrogenated Fish Oil, Hydrogenated Lard, Hydrogenated Menhaden Oil, Hydrogenated Mink Oil, Hydrogenated Olive Oil, Hydrogenated Orange Roughy Oil, Hydrogenated Palm Kernel Oil, Hydrogenated Palm Oil, Hydrogenated Peanut Oil, Hydrogenated Rapeseed Oil, Hydrogenated Shark Liver Oil, Hydrogenated Soybean Oil, Hydrogenated Sunflower Seed Oil, Hydrogenated Tallow, Hydrogenated Vegetable Oil, Isatis Tinctoria Seed Oil, Juglans Regia (Walnut) Seed Oil, Lauric/Palmitic/Oleic Triglyceride, Umnanthes Alba (Meadowfoam) Seed Oil, Unum Usitatissimum (Linseed) Seed Oil, Lupinus Albus Seed Oil, Macadamia Integrifolia Seed Oil, Macadamia Ternifolia Seed Oil, Maleated Soybean Oil, Mangifera Indica (Mango) Seed Oil, Marmot Oil, Melaleuca Alternifolia (Tea Tree) Leaf Oil, Melia Azadirachta Seed Oil, Melissa Officina lis (Balm Mint) Seed Oil, Menhaden Oil, Mink Oil, Moringa pterygosperma Seed Oil, Mortierella Oil, Neatsfoot Oil, Nelumbium Speciosum Flower Oil, Nigella Sativa Seed Oil, Oenothera Biennis (Evening Primrose) Oil, Olea Europaea (Olive) Fruit Oil, Olea Europaea (Olive) Husk Oil, Orange Roughy Oil, Orbignya Cohune Seed Oil, Orbignya Oleifera Seed Oil, Oryza Sativa (Rice) Bran Oil, Oryza Sativa (Rice) Germ Oil, Ostrich Oil, Oxidized Corn Oil, Oxidized Hazel Seed Oil, Papaver Orientale (Poppy) Seed Oil, Passiflora Edulis Seed Oil, Persea Gratissima (Avocado) Oil, Pistacia Vera Seed Oil, Placental Lipids, Prunus Amygdalus Amara (Bitter Almond) Kernel Oil, Prunus Amygdalus Dulcis (Sweet Almond) Oil, Prunus Armeniaca (Apricot) Kernel Oil, Prunus Avium (Sweet Cherry) Seed Oil, Prunus Cerasus (Bitter Cherry) Seed Oil, Prunus Persica (Peach) Kernel Oil, Pyrus Malus (Apple) Oil, Ribes Nigrum (Black Currant) Seed Oil, Ricinus Communis (Castor) Seed Oil, Rosa Canina Fruit Oil, Rosa Moschata Seed Oil, Salmon Oil, Salvia Hispanica Seed Oil, Santalum Album (Sandalwood) Seed Oil, Sesamum Indicum (Sesame) Seed Oil, Shark Liver Oil, Solanum Lycopersicum (Tomato) Seed Oil, Soybean Lipid, Sphingolipids, Taraktogenos Kurzii Seed Oil, Telphairia Pedata Oil, Vegetable Oil, Vitis Vinifera (Grape) Seed Oil, Zea Mays (Corn) Germ Oil, Zea Mays (Corn) Oil and mixtures thereof.

Non-limiting examples of suitable synthetic oils include mineral oil, isopropyl pamitate, isopropyl stearate, isohexadecane, isododecane, polyglyceryl triisostearate and mixtures thereof. Non-limiting examples of suitable silicone oils include dimethicones (including partial esters of dimethicones and fatty acids derived from natural/synthetic oils), cyclomethicones, polydimethlysiloxanes, phenyl trimethicones, trimethyl pentaphenyl trisiloxane, dimethicone copolyols and mixtures thereof. Advantageously, the hydrophobic continuous phase of the emulsion comprises dimethicone having a viscosity of 1 to 10,0000 mPa.s (centistoke), preferably 5 to 1000 mPa.s (centistoke), more preferably 25 to 500 mPa.s (centistoke).

Non-limiting examples of commercially available silicone oils include Dow Corning 200 fluid, Dow Corning 244, Dow Corning 245, Dow Corning 344, and Dow Corning 345, (commercially available from Dow Corning Corp.); SF-1204 and SF-1202 Silicone Fluids (commercially available from G.E. Silicones), GE 7207 and 7158 (commercially available from General Electric Co.); and SWS-03314 (commercially available from SWS Silicones Corp.), the Viscasil series (sold by General Electric Company), SF 1075 methyl-phenyl fluid (sold by General Electric Company) and 556 Cosmetic Grade Fluid (sold by Dow Corning Corp.), SF1066 organosilicone surfactant (sold by General Electric Company). Advantageously, where there is a choice, these oils will be selected to have a viscosity within the above-defined ranges.

The protective composition may comprise 0.1% to 15%, preferably 0.5% to 10% and more preferably 1% to 8% wax by weight of the protective composition.

As used herein, the term "wax" includes, but is not limited to, any hydrophobic material that is:

- Solid at 25°C
- practically insoluble in water according to the United States' Pharmacopeia (USP) definition in 31/NF 26 Vol. 2 General Notices, Page Xvii. (which, according to that definition, means that more than 10,000 parts of water are needed to dissolve 1 part solute);
- has an onset temperature measured according to the DSC Method, defined hereinbelow, which is 10°C or greater; and
- comprises lipids, silicones or mixtures thereof.

The wax may comprise natural wax, synthetic wax, silicone wax, or mixtures thereof.

Non-limiting examples of suitable natural waxes include Abies Alba Leaf Wax, Acacia Dealbata Leaf Wax, Acacia Farnesiana Flower Wax, Beeswax, Ceresin, Cetyl Esters, Cistus Labdaniferus Flower Wax, Aurantium Amara (Bitter Orange) Flower Wax, Aurantium Dulcis (Orange) Peel Wax, Copernicia Cerifera (Carnauba) Wax, Eclipta Prostrata Wax, Euphorbia Cerifera (Candelilla) Wax, Helichrysum Angustifolium Wax, Jasminum Officina le (Jasmine) Flower Wax, Jasminum Sambac (Jasmine) Flower Wax, Jojoba Esters, Jojoba Wax, Lanolin Wax, Lavandula Angustifolia (Lavender) Flower Wax, Lawsonia Inermis Wax, Mink Wax, Montan Acid Wax, Montan Wax, Myrica Cerifera (Bayberry) Fruit Wax, Ocimum Tenuiflorum Wax, Olive Wax, Oryza Sativa (Rice) Bran Wax, Ouricury Wax, Palm Kernel Wax, Persea Gratissima (Avocado) Wax, Pistacia Lentiscus Leaf Wax, Polianthes Tuberosa Flower Wax, Pyrus Malus (Apple) Peel Wax, Ribes Nigrum (Black Currant) Wax, Rosa Centifolia Flower Wax, Salvia Sclarea (Clary) Wax, Shellac Wax, Simmondsia Chinensis (Jojoba) Butter, Soft Olive Wax, Spent Grain Wax, Stipa Tenacissima Wax, Sunflower Seed Wax, Vegetable Wax, Vitis Vinifera (Grape) Leaf Wax and mixtures thereof.

Non-limiting examples of suitable synthetic waxes include Hydrogenated Japan Wax, Hydrogenated Jojoba Oil, Hydrogenated Jojoba Wax, Hydrogenated Microcrystalline Wax, Hydrogenated Rice Bran Wax, Hydrolyzed Beeswax, Microcrystalline Wax, Oxidized Beeswax, Oxidized Microcrystalline Wax, Ozokerite, Paraffin, PEG-6 Beeswax, PEG-8 Beeswax, PE G-12 Beeswax, PEG-20 Beeswax, PEG-12 Carnauba, Potassium Oxidized Microcrystalline Wax, Sulfurized Jojoba Oil, Synthetic Beeswax, Synthetic Candelilla Wax, Synthetic Carnauba, Synthetic Japan Wax, Synthetic Jojoba Oil, Synthetic Wax and mixtures thereof.

Non-limiting examples of suitable silicone waxes include DC2503 Cosmetic Wax, DC580 wax, DC AMS-C30 Cosmetic Wax, C30-45 Alkyl Methicone, DC Silkywax 10, Hexamethyldisiloxane, DC ST-Wax 30, C30-45 Alkyldimethylsilyl Polypropylsilsesquioxane, DC SW-8005 resin wax, C26 - 28 Alkyl Dimethicone, C26 - 28 Alkyl Methicone, Polyphenylsilsesquioxane and mixtures thereof.

Advantageously, the wax comprises beeswax, carnauba wax, candelilla wax, jojoba waxvegetabe wax, ozokerite, arachidyl behenate, or mixtures thereof.

As demonstrated by the Franz Cell data below, the presence of some wax in the hydrophobic continuous phase of the protective composition may surprisingly further reduce penetration by thioglycolic acid in comparison with oils alone, while still allowing hair removal. Without wishing to be bound by theory, applicants believe that this additional effect may be because the wax militates against the tendency otherwise exhibited by oils to ball up on skin and therefore disrupt the barrier. The presence of wax may also ensure that a thin barrier of the protective composition can be evenly distributed across the skin, even at a low dosage per unit area. The wax may form a substantive barrier across the skin (enhanced by an amorphous mix of the oil & wax) that is chemically resistant to ingress from the thioglycolate (or other reducing) actives, therefore physically reducing the ability for the harsh chemistry to come into contact with the skin. This reduction in contact means that the stratum corneum may be maintained in a better state than if no barrier were present with correspondingly reduced signs of irritation, such as erythema, tingling and stinging.

At the same time as reducing contact between the depilatory active ingredient and the skin, the present compositions still permit the depilatory composition to attack and degrade the unwanted hair growing on that skin to achieve hair removal. Why this should be is not fully understood, but it may simply be due to the fact that less of the protective composition adheres to the hairs than to the skin.

The hydrophobic continuous phase may comprise one or more triglycerides, the or each triglyceride having the following formula:

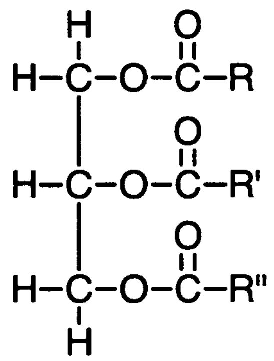

wherein R, R' and R" may be the same as or different from one or both of the others, wherein each of R, R' and R" is a fatty acid and wherein the or each triglyceride is solid at 25°C

Advantageously, the or each triglyceride has an onset temperature of less than 65°C as measured by Differential Scanning Calorimetry. At and above an onset temperature of 65°C, the composition may become increasingly difficult to apply and may, in addition, crack and fall off in use.

Suitable oils from which triglycerides may be formed from include, but are not limited to, the oils listed herein. Suitable fatty acids for formation of triglycerides include, but are not limited to, Myristoleic acid, Palmitoleic acid, Sapienic acid, Oleic acid, Linoleic acid, $\alpha$-Linolenic acid ,Arachidonic acid , Eicosapentaenoic acid, Docosahexaenoic acid, Lauric acid ($C_{12}$), Myristic acid ($C_{14}$), Palmitic acid ($C_{16}$), Stearic acid ($C_{18}$), Arachidic acid ($C_{20}$) and mixtures thereof.

Specific sources of triglycerides suitable for inclusion in the protective composition include include Butter, Shea Butter, Butyrospermum Parkii, Lipex Shea, Theobroma Cacao (Cocoa) Seed Butter, Cocoa Butter, Hydrogenated Shea Butter, Hydrogenated Cocoa Butter, Irvingia Gabonensis Kernel Butter, Tallow, Lard, Mangifera Indica (Mango) Seed Butter, Kokum Butter and mixtures thereof. The triglyceride(s) may fall under the definition of "wax" or "oil" as used herein and, in such a case, should be included as a wax or oil for the purposes of determining the proportions of wax or oil.

The hydrophobic continuous phase may comprise skin active agents such as, but not limited to oil soluble vitamins, such as vitamin E derivatives, including vitamin E acetate and tocopherol nicotinate; oil-soluble vitamin A derivatives, such as retinyl palmitate; lanolin; ceramides; sterols and sterol esters; salicylic acid; camphor; eucalyptol; essential oils and mixtures thereof. These materials may fall under the definition of "wax" or "oil" as used herein and, in such a case, should be included as a wax or oil for the purposes of determining the proportions of wax or oil.

The protective composition used in the method and comprised within the kit according to the invention may include further ingredients such as, but not limited to metal oxides, organic and inorganic dyes, lakes, micas, flavourings, perfumes and mixtures thereof.

The protective composition used in the method and comprised within the kit according to the invention comprises an emulsion having a hydrophilic dispersed phase. Advantageously, the protective composition comprises from 4.9% to 89.9%, preferably 10% to 75%, more preferably 25% to 65% hydrophilic dispersed phase by weight of the protective composition.

The hydrophilic dispersed phase typically comprises water, but need not. In addition to or as an alternative to water, the hydrophilic dispersed phase may comprise hydrophilic materials, such as polyhydric alcohols, ethoxylated and propoxylated polyols, polysaccharides, and mixtures thereof. Suitable polyhydric alcohols (polyols) include, but are not limited to, butylene glycol, hexylene glycol, ethoxydiglycol, dipropylene glycol, phenyl ethyl alcohol, glycerin, 1,3-butanediol, 1,2-propanediol, isoprene glycol, sorbitol, polyethylene glycol, polypropylene glycol and mixtures thereof. Preferred polyols include glycerin, propylene glycol, panthenol and mixtures thereof.

Additionally, the hydrophilic dispersed phase may comprise other polar solvents, such as alcohols, ketones and mixtures thereof. Examples of suitable polar solvents include phenyl ethyl alcohol, ethanol, isopropyl alcohol and mixtures thereof.

The hydrophilic dispersed phase may additionally comprise hydrophilic skin active agents such as, but not limited to, vitamins, including hydrophilic ascorbic acid compounds and vitamin B3 compounds; azelaic acid; gallic acid and its derivatives; N-acetyl glucosamine; panthenol and mixtures thereof.

The protective compositions used in the method and comprised within the kit according to the invention comprises an emulsifier. The emulsifier, or, if more than one emulsifier is present, then combination of emulsifiers, has an Hydrophilic-Lipophilic Balance (HLB) from 1 to 8, preferably from 1.5 to 7 and more preferably from 3 to 6. The HLB (the "Hydrophilic-Lipophilic Balance") value system is fully described, and values for various materials are provided, in the publication The HLB System, A Time-Saving Glide to Emulsifier Selection (published by ICI Americas Inc., Wilmington, Del.; 1984).

The protective composition may comprise from 0.1% to 10%, and preferably from 0.1% to 5% emulsifier by weight of the protective composition.

The emulsifier may be nonionic, anionic or cationic. Suitable emulsifiers are disclosed in McCutcheon's Detergents and Emulsifiers, North American Edition, pages 317-324 (1986). Illustrative nonionic surfactants are alkoxylated compounds based on $C_{10}$ - $C_{22}$ fatty alcohols and acids, and sorbitan, available as the following, commercial products:

- Products sold under the Neodol® trademark and manufactured by the Shell Chemical Company;
- Copolymers of polyoxypropylene-polyoxyethylene, sold by the BASF Corporation under the Pluronic® trademark
- Alkyl polyglycosides available from the Henkel Corporation.

Anionic emulsifiers or surfactants which may be used according to the invention include fatty acid soaps and synthetic detergents, such as sodium lauryl sulphate, sodium lauryl ether sulphate, alkyl benzene sulphonate, mono-and di-alkyl acid phosphates and sodium fatty acyl isethionate. Amphoteric emulsifiers or surfactants according to the invention include as dialkylamine oxide and betaines, such as cocamidopiopyl betaine.

If the hydrophobic continuous phase comprises silicones, the emulsifiers are preferably selected from polyoxyalkylene copolymers, polyglyceryl copolymers or mixtures thereof. Commercially available examples of polyoxyalkylene copolymers include DC5225C or DC2-5185C (PEG/PPG-18/18 dimethicone available as blend with cyclopentasiloxane) from Dow Corning Corp. and KF6017, KF6028 (PEG-9 dimethicone) or KF6038 from Shin-Etsu Inc. A commercially available example of polyglyceryl emulsifiers include KF6100 and KF6104 from Shin-Etsu Inc. and Abil WE-09 and Abil

EM90 from Evonik Industries.

Advantageously, the keratin reducing agent is comprised within the depilatory composition in an amount from 0.3% to 20%, preferably from 0.8% to 15%, more preferably from 1% to 10% by weight of the depilatory composition.

The depilatory composition comprises at least one thioglycolate salt or thioglycollic acid acting as a hair removal agent when the depilatory composition is applied to unwanted hair. Preferably, the depilatory composition comprises sodium, potassium, magnesium, calcium, beryllium, strontium, zinc, monoethanolamine, ammonium, tetralkylammonium, imidazolium, pyridinium, phosphonium or glyceryl thioglycolate salts, or mixtures thereof, which may include dianion forms of thioglycolate. More preferably, the depilatory composition comprises at least one of sodium, potassium, magnesium or calcium thioglycolate, or mixtures thereof. Even more preferably the depilatory composition comprises potassium or calcium thioglycolate, or mixtures thereof.

The pH of the depilatory composition may advantageously be in the range of from 6 to 13.8, preferably from greater than 7 to 13, more preferably from 9 to 12.9, even more preferably from 10 to 12.8, even more preferably still from 12 to 12.75 and yet more preferably from 12.3 to 12.6 to improve the efficacy of the active ingredient. The depilatory composition may, in a preferred embodiment, comprise at least one base to control the pH. Preferably, the depilatory composition comprises potassium hydroxide; sodium hydroxide; lithium hydroxide; calcium hydroxide; barium hydroxide; caesium hydroxide; sodium hydroxide; ammonium hydroxide; strontium hydroxide; rubidium hydroxide; magnesium hydroxide; zinc hydroxide; sodium carbonate; pyridine; ammonia; alkanolamides (including monoethanolamine, diethanolamine, triethanolamine), phosphates (including tetrasodium phosphate), arginine or mixtures thereof. More preferably, the depilatory composition comprises at least one buffering base, even more preferably the depilatory composition comprises calcium hydroxide, magnesium hydroxide; barium hydroxide; strontium hydroxide; zinc hydroxide; arginine or mixtures thereof. Still more preferably the depilatory composition comprises calcium hydroxide; magnesium hydroxide, zinc hydroxide, sodium hydroxide, potassium hydroxide or mixtures thereof. Even more preferably still, the depilatory composition comprises calcium hydroxide, sodium hydroxide or mixtures thereof.

In an advantageous embodiment, the base is present at a concentration of from 0.1% to 10.0%, more preferably from 0.5% to 8.0% and even more preferably from 1.0% to 5.0%, by weight of the depilatory composition.

The concentration of water in the depilatory composition is preferably at least 40%, more preferably from 50% to 98%, even more preferably from 60% to 95% and even more preferably still from 70% to 90%, by weight of the depilatory composition.

The depilatory composition may optionally comprise a thickening agent. A representative but not exhaustive list can be found in "The Encyclopaedia of Polymers and Thickeners for Cosmetics" compiled and edited by Robert Y. Lochhead, PhD and William R. Fron, Department of Polymer Science, University of Southern Mississippi. Exemplary classes of thickening agents include gums, carbomers, polymers and copolymers of acrylic acid, associated thickeners, layered silicates/clays and natural polymers (including polysaccharides). One or more thickening agents may be included in the aqueous depilatory composition. The thickening agent may be present at a level of from about 0.01% to about 20%, preferably from about 0.1% to about 10% by weight of the depilatory composition.

The depilatory composition may also include other skin care ingredients such as conditioning agents selected from the group consisting of humectants, moisturizers, or skin conditioners (including mineral oil; almond oil; chamomile oil; jojoba oil; avocado oil; shea butter, niacinamide and glycerine); skin rejuvenation compositions (for example targeted for fine lines, wrinkles and uneven skin tone, including retinoids), cosmetic compositions; anti-inflammatory agents (including corticosteroids); anti-oxidants (including flavonoids) radical scavengers; sunscreen agents; skin cooling or warming agents and the like. The depilatory composition may comprise one or more skin care ingredients present in an amount of from about 0.001% to about 10%, more preferably from about 0.01% to about 7%, and even more preferably from about 0.025% to about 5%, by weight of the depilatory composition.

An accelerant may be employed in the depilatory composition. This optional component accelerates the rate of depilatory action of the depilatory agent. Suitable accelerants include, but are not limited to, urea; thiourea; dimethyl isosorbide; arginine salts; ethoxydiglycol; propylene glycol and methylpropyldiol. The accelerant may be present in a concentration range of from 0.5% to 10%, more preferably from 2% to 8% and even more preferably from 2% to 5% by weight of the depilatory composition.

The depilatory composition may further comprise components known, conventionally used, or otherwise effective for use in cosmetic compositions, such as dyes; pigments (including ultra marines and talc); anionic, cationic, nonionic and/or amphoteric or zwitterionic surfactants, polymers (including hydrophobically modified polymers); dispersing agents; solvents; lubricants; fragrances; preservatives; chelants, proteins and derivatives thereof, plant materials (e.g. aloe, chamomile and henna extracts); silicones (volatile or non-volatile, modified or non-modified); film-forming agents; film forming promoters and mixtures thereof.

The depilatory composition may be formulated in any common delivery form, such as a cream or lotion. Alternatively, it may be delivered on a substrate, such as a thin film of depilatory composition coated onto the substrate. The substrate may be configured in any suitable form, such as a strip, mask or patch.

In addition to the protective composition and the depilatory composition, the kit according to the second aspect

of the invention may comprise one or more of:

(a) A make-up removal composition and/or a make-up removal wipe;

(b) Means for removal of the protective composition and the depilatory composition following use, which means may comprise one or more of a tool, such as a scraper or a spatula; or a wipe;

(c) A post-treatment composition skin care composition to be applied to the area of skin from which hair has been removed. Such a post-treatment skin care composition may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like. The post-treatment skin care composition may be leave-on or a rinse-off composition.

(d) Instructions regarding how to use the various elements of the kit, which instructions may comprise one or more elements of the method as defined herein.

Prior to applying the method or using the kit according to the present invention, a user should advantageously remove all make-up from the skin, to ensure good adherence and effective application of both the protective composition and the depilatory composition.

The method according to the first aspect of the invention comprises the step of applying the above-defined protective composition to an area of skin on which unwanted hair is growing, which may be located on any part of the human body.

Advantageously, the protective composition is not just applied to the area to be depilated, but also to an immediately juxtaposing area thereabout (that is, the protective composition is applied to an area of skin which is greater than just the area which is to be depilated).

Advantageously, the user will apply from 0.3 - 2mg of protective composition per square centimetre of skin, preferably from 0.4 - 1.3mg/cm$^2$, more preferably from 0.4 to 1mg/cm$^2$. Following application, the protective composition is advantageously massaged into the skin. Preferably, massaging is effected for at least 10 seconds, and, more preferably, massaging is effected as a circular motion. Without wishing to be bound by theory, it is believed that the protective composition may trap hair within it thereby shielding it from the to-be-applied depilatory composition; massaging may help to release the hairs from the skin and ensure improved access thereto by the depilatory composition.

The method according to the first aspect of the invention comprises the subsequent step of applying the above-defined depilatory composition to an area of skin on which unwanted hair is growing and to which protective composition has already been applied. Advantageously, the user will apply a layer of depilatory composition which is from 0.1 to 5mm, preferably from 0.3 to 3mm, more preferably from 0.5 to 2mm in thickness.

Subsequently, according to the method of the first aspect of the invention, the depilatory composition is advantageously left in place for at least 1 minute, preferably from 1 to 10 minutes, more preferably from 3 to 10 minutes, depending on the thickness of the hair and the hair removal efficacy of the depilatory composition (which, in turn, is dependent upon the concentration of keratin reducing agent in the depilatory composition).

Subsequently, according to the method of the first aspect of the invention, the protective composition and the depilatory composition are advantageously removed. This may be achieved using one or more of a cotton wool ball, pad or wand, a tissue, a cloth, or a tool, such as a spatula or a scraper. Advantageously, the skin from which hair has been removed is then rinsed with water.

In an advantageous subsequent step, a post-treatment skin care composition may be applied to the area of skin from which hair has been removed. Such a post-treatment skin care composition may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like. The post-treatment skin care composition may be leave-on or a rinse-off composition.

<u>Differential Scanning Calorimetry (DSC) Melting Method</u>

This method is the American Oil Chemists' Society Method Cj 1-94, as reapproved in 2009 and it determines the "onset temperature" (that is the temperature of onset of melting) of oils and fats by differential scanning calorimetry (DSC).

<u>Apparatus</u>

1. Aluminum capsules.

2. DSC instrument, capable of holding temperature at -60°C and achieving a temperature of 80°C,

Reagents

1. Indium, powder 60 mesh, 99.999%, such as Aldrich Chemical Co., Milwaukee, WI 53233, or equivalent.

2. *n*-Decane, 99+%, such as Aldrich Chemical Co., Milwaukee, WI 53233, or equivalent.

3. Methyl stearate, 99%, such as Aldrich Chemical Co., Milwaukee, WI 53233, or equivalent.

Procedure

1. Standardization of equipment—Proceed with the normal standardization using both indium and n-decane as reference standards. Follow instrument manual for adjustment to lock onto these two reference points and flatten the baseline slope as much as possible when empty pans are analyzed. Analyze the secondary standard (methyl stearate). Weigh 5 mg of the standard into the same kind of pan which will be used for the test portion (if hermetically sealed, it may be reused at a later date). Use the method sequence in Procedure, 2-7 to obtain the melting point onset (because of the high purity, only a 2 min hold is necessary for the standard after crystallization). Be certain that the heating rate during the definitive heating pattern is at 5°C/min. The melting point onset should be within ±2.00°C of 36.5°C. If not, recheck calibration.
*Note-be* certain to use identical capsules for the test portion as those used for reference standards and the instrument blank reference.

2. Melt each test portion completely and weigh 7 ± 0.200 mg of each test portion into the same kind of capsule used for the blank and reference samples (aluminum) and seal to minimize oxidation and other changes.

3. Place capsules in DSC at room temperature.

4. Heat rapidly to 80°C and hold for 10 min.

5. Cool to -60°C at 10°C/min and hold for 30 min.

6. Heat to 80°C at 5°C/min.

7. Use the baseline obtained for an empty capsule analysis from the final melt segment of the program to define the position of the baseline under the sample peaks. Overlay the final melting curve of the test portion over the curve for the empty capsule with a flexible ruler or other curve guide to define the baseline of the test portion back to where it intersects the initial deviation of the melting curve from its baseline. The baseline beneath the test portion should be a continuation of the baseline where there are no sample components present. If a shift has occurred in the heat capacity of the test portion after the melt, it will be evident relative to the baseline of the empty capsule. Have the instrument calculate the sigmoid baseline if it can, or connect the end of the peak point with the last point in which the test portion was in conjunction with the baseline of the empty capsule.

Results

Determine the onset temperature in °C, which, if not computer generated, is an extrapolation to baseline of the steepest slope of the principal peak.

Franz cell method

Principle and Scope:

This method is applicable for using Franz cell apparatus for the *in-vitro* assessment of penetration of thioglycolic acid (TGA) and its salts through a skin mimic after the application of a depilatory composition following pre-treatment with a protective composition.

Penetrated TGA is quantified using Reverse Phase High Performance (or Pressure) Liquid Chromatography (RP-HPLC) with external standard quantitation at 240nm.

Method

Reference is made to Figure 1 and to the reference numerals therein:

1. Prepare the Vitro-Skin (IMS Vitro-Skin®, Catalogue number: P&G1013, made by IMS Inc., Portland, Maine, USA) samples by cutting 8x6.2cm segments and placing them textured side up on the racks into a hydration chamber (manufactured & sold by IMS) containing a 14.7% glycerol solution. The hydration chamber should be sealed and the vitro-skin left to hydrate at room temperature and a humidity of 80.4% ±3.5% for 24 hours.

2. Prepare the receptor solution for the Franz-cell by mixing 1.90ml formic acid (98%wt+ Fluka, by Sigma Aldrich, or equivalent), 30ml acetonitrile (RP-HPLC grade) and 968.1ml water (RP-HPLC grade). Set up the static Franz cell (Permegear or equivalent, 15mm diameter unjacketed cell with a 12ml receptor volume) by clamping it in place over suitable stirrer plates (not shown) and add a small stirrer bar (6) to each cell, fill the receptor cell (2) to the brim with the required amount of receptor solution.

3. Once hydrated, remove a sheet of vitro-skin from the hydration chamber and lay textured side up on a clean flat surface then dose 100μl (~2mg/$cm^2$) of protective composition (not shown) onto the vitro-skin and spread evenly over the surface by rubbing for 30 seconds with a gloved finger.

4. Using a scalpel blade cut the vitro-skin segment (3) into two equal sections, each large enough to completely cover the top of the cell. Place the relevant size o-ring (5) (22mm, for the specified Franz-cell) onto each section of the vitro skin and dose to 150mg/$cm^2$ of depilatory composition (4) ("Veet Normal Skin Hair Removal Cream" or an equivalent (an equivalent being a composition comprising 3.7%wt thioglycolic acid)) into the centre then, using a glass rod, evenly spread the cream around the inside of the o-ring (5). Using tweezers pick up the vitro-skin segment and place the vitro-skin segment, depilatory and o-ring centrally over the receptor cell (2), place donor cell (1) over the top and clamp in place. Turn on stirrer plate and start 10 minute countdown timer. After 10 minutes; turn off stirrer and remove the clamp, donor cell (1) and vitro-skin segment and place the receptor solution in a suitable container for analysis.

5. A reference sample should also be run without protective composition treatment on the vitro-skin. Remove a sheet of vitro-skin from the hydration chamber and lay textured side up on a clean flat surface. Repeat step 4 of the protocol to produce the reference sample.

Sample Analysis

For RP-HPLC analysis, prepare a 50mM Formic acid (98%+ Fluka) solution and mix 970ml of this solution with 30ml acetonitrile (HPLC grade) to act as a mobile phase during the analysis.

A reference standard solution should be made with a concentration of Calcium Thioglycolate Trihydrate of 0.94 mg/ml.

Install a Waters Atlantis T3 3μm 4.6 x 50mm column into the HPLC (although any silica- based $C_{18}$ reversed phase RP-HPLC column may be used), and ensure all solvent lines for the RP-HPLC are primed and free of leaks. Allow the mobile phase to circulate through the system for 25 minutes at 0.7mL/Min in order to equilibrate the column. Detection of the thioglycolic acid is via UV spectroscopy.

The RP-HPLC conditions are as follows:

- Injection volume: 20 μL
- Mobile phase flow rate: 0.70 ml/min
- Run time: 10 minutes

- UV Detection wavelength: 240 nm
- Column temperature: 35°C
- UV sampling rate: ≥ 5 per second
- Retention time: Thioglycolic Acid ~ 2.5 min

Calculations:

Calculate the concentration of Thioglycolic Acid in the sample

$$\text{concentration (mg/ml)} = \frac{\textbf{weight of std (mg) x purity}}{\textbf{25}} \times \frac{\textbf{3}}{\textbf{25}}$$

Calculate the concentration of thioglycolic acid in the sample using the following formula:

$$\text{concentration (mg/ml)} = \textbf{A/B x C x E/F}$$

Where,

A = Peak Area of Thioglycolic Acid Sample
B = Average Peak Area of Thioglycolic Acid Standard
C = Thioglycolic Acid final STD concentration in mg/ml (0.94 mg/ml)
E = Molecular weight of Thioglycolic acid (92.12g/mol)
F = Molecular weight of Calcium thioglycolate (184.23g/mol)

The efficacy of the barrier provided by the protective e composition (resistance to TGA penetration) can be calculated as a percentage decrease in TGA in the receptor solution:

$$\%\text{reduction} = \frac{\text{concentration without barrier* – concentration with barrier*}}{\text{concentration without barrier*}} \times 100$$

*protective composition

For example, if TGA in solution without protective composition = 75μg/ml and TGA in solution with barrier = 15 μg/ml

$$\%\text{reduction} = \frac{75 - 60}{75} \times 100 = \underline{\textbf{85\%}}$$

A reduction of TGA penetration of 45% or more is believed to correlate to a significant and user-noticeable reduction in irritation.

Examples

Inventive Example 1

Premix A

| INCI Name | Trade Name | %w/w |
|---|---|---|
| Cyclopentasiloxane | DC245 | 1.000 |
| Trihydroxystearin | Thixcin R | 0.300 |

Main Mixture

| INCI Name | Trade Name | %w/w |
|---|---|---|
| Cyclopentasiloxane | DC245 | 39.550 |
| Cyclopentasiloxane & Dimethicone Copolyol Polyglyceryl-4 Isostearate; Cetyl PEG/PPG-10/1 | DC2-5185C | 4.700 |
| Dimethicone; Hexyl Laurate | Abil WE09 | 0.200 |
| Arachidyl Behenate | Arachidyl Behenate | 2.000 |
| Dimethicone 50cs | DC200 50cs | 3.000 |
| Dimethicone 350cs | DC200 350cs | 2.000 |

Premix B (Water Phase)

| INCI Name | Trade Name | %w/w |
|---|---|---|
| Purified water | Deionised Water | 45.250 |
| Sodium Chloride Extra Pure | Sodium Chloride | 2.000 |

The composition of Inventive Example 1 was tested using a Franz Cell according to the above-defined method and gave a percentage reduction in thioglycolate penetration of 95%.

Making Instructions

- Add all ingredients from Premix A into a premix tank (PMT1) and stir using a spatula to ensure that no lumps are present.
- Leave the contents of PMT1 to thicken at room temperature for one hour.
- Add all ingredients from Premix B into a premix tank (PMT2) and stir using a spatula until fully dissolved.
- Place all ingredients from the Main Mixture into the main mixing tank (MMT). Heat to 86°C using a water bath, whilst stirring constantly using an overhead stirrer set to ~300rpm. Ensure enough agitation to stop the wax from settling to the bottom of the MMT.
- Once the ingredients within the MMT have reached 86°C, maintain stirring and leave the MMT at 86°C for 45 minutes to ensure that the hydrophobic continuous phase has fully melted.
- Using a high shear mixing device (such as a Silverson Laboratory Mixer available from Silverson Machines), mix the contents of the MMT for 5 minutes at ~7000rpm to ensure full dispersion of waxes within the mixture.
- Cool the MMT to room temperature whilst mixing at ~300rpm with the high shear mixer. At room temperature transfer Premix A from PMT1 to the MMT & homogenise using the mill for 2 minutes at 3000rpm to ensure the hydrophobic continuous phase is fully homogenous. • Transfer the Water Phase (C) from PMT2 to the MMT. Mix together the water and hydrophobic continuous phases to form the water in oil emulsion using the high shear mixer set to 7000rpm for 10 minutes.

The compositions of Inventive Examples 2-6 were made in an analogous fashion to Inventive Example 1. The compositions were then tested using the Franz Cell method defined above.

| Example | Protective Pomposition | % Reduction in Thioglycolic Acid Penetration According to the Franz Cell Method |
|---|---|---|
| | 45.25% Water | |

(continued)

| Example | Protective Pomposition | % Reduction in Thioglycolic Acid Penetration According to the Franz Cell Method |
|---|---|---|
| Inventive Example 2 | 40.55% Cyclopentasiloxane<br>3% Dimethicone (50cSt)<br>2% Dimethicone (350cSt)<br>4.7% Cyclopentasiloxane & Dimethicone Copolyol<br>2% Sodium Chloride<br>2% Arachidyl Behenate (Wax)<br>0.3% Trihydroxystearin<br>0.2% Polyglyceryl-4 Isosterarate & Cetyl Dimethicone Copolyol & Hexyl Laurate | 95 |
| Inventive Example 3 | 55.25% Water<br>30.92% Cyclopentasiloxane<br>3% Dimethicone (50cSt)<br>2% Dimethicone (350cSt)<br>4.7% Cyclopentasiloxane & Dimethicone Copolyol<br>2% Sodium Chloride<br>1.63% Arachidyl Behenate (Wax)<br>0.3% Trihydroxystearin<br>0.2% Polyglyceryl-4 Isosterarate & Cetyl Dimethicone Copolyol & Hexyl Laurate | 95 |
| Inventive Example 4 | 65.25% Water<br>21.28% Cyclopentasiloxane<br>3% Dimethicone (50cSt)<br>2% Dimethicone (350cSt)<br>4.7% Cyclopentasiloxane & Dimethicone Copolyol<br>2% Sodium Chloride<br>1.27% Arachidyl Behenate (Wax)<br>0.3% Trihydroxystearin 0.2% Polyglyceryl-4 Isosterarate & Cetyl Dimethicone Copolyol & Hexyl Laurate | 94 |
| Inventive Example 5 | 37.25% Water<br>45.55% Cyclopentasiloxane<br>8% Propylene Glycol<br>4.7% Cyclopentasiloxane & Dimethicone Copolyol<br>2% Sodium Chloride<br>2% Arachidyl Behenate (Wax)<br>0.3% Trihydroxystearin<br>0.2% Polyglyceryl-4 Isosterarate & Cetyl Dimethicone Copolyol & Hexyl Laurate | 60 |
| Inventive Example 6 | 45.25% Water<br>45.75% Mineral Oil<br>5% ShinEtsu KF-6038 (Lauryl PEG-9 Polymethylsiloxyethyl Dimethicone)<br>2 % Sodium Chloride<br>2% Arachidyl Behenate (Wax) | 68 |
|  | 45.25% Water |  |

(continued)

| Example | Protective Pomposition | % Reduction in Thioglycolic Acid Penetration According to the Franz Cell Method |
|---|---|---|
| Inventive Example 7 | 40.55% Cyclopentasiloxane<br>3% Dimethicone (50cSt)<br>2% Dimethicone (350cSt)<br>4.7% Cyclopentasiloxane & Dimethicone Copolyol<br>2% Sodium Chloride<br>2% Sumac Wax<br>0.3% Trihydroxystearin<br>0.2% Polyglyceryl-4 Isosterarate & Cetyl Dimethicone Copolyol & Hexyl Laurate | 80 |
| Inventive Example 8 | 45.25% Water<br>47.75% Mineral Oil<br>5% ShinEtsu KF-6038 (Lauryl PEG-9 Polymethylsiloxyethyl Dimethicone)<br>2% Sodium Chloride | 50 |
| Inventive Example 9 | 47.25% Water<br>29.50% Cyclopentasiloxane<br>8.00% Propylene Glycol<br>4.7% Cyclopentasiloxane & Dimethicone Copolyol<br>3% Dimethicone (50cSt)<br>2% Dimethicone (350cSt)<br>2% Sodium Chloride<br>2% Arachidyl Behenate (Wax)<br>0.50% Benzyl Alcohol<br>0.45% Phenoxyethanol<br>0.3% Trihydroxystearin<br>0.2% Polyglyceryl-4 Isosterarate & Cetyl Dimethicone Copolyol & Hexyl Laurate<br>0.1% Sodium Benzoate | 80 |
| Comparative Example 1 | 100% mineral oil | 25.0 |
| Comparative Example 2 | 100% Sunflower Seed oil | 10.8 |
| Comparative Example 3 | 100% olive oil | 0.0 |

As demonstrated by the Franz Cell data, oils, on their own (see Comparative Examples 1, 2 and 3) provide little to no barrier to thioglycolic acid, whereas the emulsion systems present a dramatically superior barrier to penetration (see Inventive Examples 1-9).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A method of removing hair from skin comprising the steps of:

(a) applying a protective composition to an area of skin on which unwanted hair is growing, wherein the protective composition comprises an emulsifier having a Hydrophilic-Lipophilic balance from 1 to 8 and an emulsion having a hydrophobic continuous phase and a hydrophilic dispersed phase;
(b) applying a depilatory composition to the area of skin to which the protective composition has been applied, the depilatory composition comprising a keratin reducing agent, the keratin reducing agent comprising at least one thioglycolate salt or thioglycolic acid.

**2.** The method of claim 1, wherein the emulsion comprises 10% to 95%, preferably, 15% to 95%, more preferably 25% to 75% and more preferably still 35% to 65% hydrophobic continuous phase by weight of the protective composition.

**3.** The method of claim 1 or 2, wherein the hydrophobic continuous phase comprises wax.

**4.** The method of any of claims 1 to 3, wherein the protective composition comprises 0.1% to 15%, preferably 0.5% to 10%, more preferably 1% to 8% wax by weight of the protective composition.

**5.** The method of claim 3 or 4, wherein the wax comprises natural wax, synthetic wax, silicone wax, or mixtures thereof.

**6.** The method of any preceding claim, wherein the hydrophobic continuous phase comprises silicone oil, preferably dimethicone having a viscosity of 1 to 10,0000 mPa.s (centistoke), preferably 5 to 1000 mPa.s (centistoke), more preferably 25 to 500 mPa.s (centistoke).

**7.** The method of any preceding claim, wherein the emulsifier has a Hydrophilic-Lipophilic Balance from 1.5 to 7 and more preferably from 3 to 6.

**8.** The method of any preceding claim, comprising the following additional step between step (a) and step (b):

(a1) massaging the protective composition into the skin for at least 10 seconds.

**9.** The method of any preceding claim, comprising the following additional step immediately following step (b):

(c) leaving the depilatory composition in place on the protective composition for a period of at least 1 minute, preferably from 1 to 10 minutes, more preferably from 3 to 10 minutes.

**10.** The method of claim 9, comprising the following additional step immediately following step (c):

(d) removing both the protective composition and the depilatory composition from the skin, optionally scraping, wiping or rubbing it off.

**11.** The method of any preceding claim, wherein the thioglycolate salt comprises potassium or calcium thioglycolate, or mixtures thereof.

**12.** A depilatory kit comprising:

(a) a protective composition, the protective composition comprising an emulsifier having a Hydrophilic-Lypophilic balance of from 1 to 8 and an emulsion having a hydrophobic continuous phase and a hydrophilic dispersed phase;
(b) a depilatory composition comprising an effective amount of a keratin reducing agent, the keratin reducing agent comprising at least one thioglycolate salt or thioglycolic acid.

**13.** The depilatory kit of claim 12, wherein protective composition comprises 0.1% to 15%, preferably 0.5% to 10%, more preferably 1% to 8% wax by weight of the protective composition.

**14.** The depilatory kit of claims 12 or 13, additionally comprising a tool, such as a scraper or a spatula, or a wipe.

**Patentansprüche**

**1.** Verfahren zum Entfernen von Haaren von der Haut, umfassend die Schritte:

(a) Auftragen einer Schutzzusammensetzung auf einen Bereich der Haut, in dem unerwünschte Haare wachsen, wobei die Schutzzusammensetzung einen Emulgator mit einem HLB-Wert (hydrophile und lipophile Anteile) von 1 bis 8 und eine Emulsion mit einer hydrophoben kontinuierlichen Phase und einer hydrophilen dispergierten Phase aufweist,
(b) Auftragen einer Enthaarungszusammensetzung auf den Bereich der Haut, auf den die Schutzzusammensetzung aufgetragen wurde, wobei die Enthaarungszusammensetzung ein Keratinreduktionsmittel umfasst, wobei das Keratinreduktionsmittel mindestens ein Thioglycolatsalz oder Thioglycolsäure umfasst.

**2.** Verfahren nach Anspruch 1, wobei die Emulsion zu 10 Gew.-% bis 95 Gew.-%, vorzugsweise zu 15 Gew.-% bis 95 Gew.-%, mehr bevorzugt zu 25 Gew.-% bis 75 Gew.-% und noch mehr bevorzugt zu 35 Gew.-% bis 65 Gew.-% der Schutzzusammensetzung hydrophobe kontinuierliche Phase umfasst.

**3.** Verfahren nach Anspruch 1 oder 2, wobei die hydrophobe kontinuierliche Phase Wachs umfasst.

**4.** Verfahren nach einem der Ansprüche 1 bis 3, wobei die Schutzzusammensetzung zu 0, Gew.-% bis 15 Gew.-%, vorzugsweise zu 0,5 Gew.-% bis 10 Gew.-%, mehr bevorzugt zu 1 Gew.-% bis 8 Gew.-% der Schutzzusammensetzung Wachs umfasst.

**5.** Verfahren nach Anspruch 3 oder 4, wobei das Wachs natürliches Wachs, synthetisches Wachs, Silikonwachs oder Mischungen davon umfasst.

**6.** Verfahren nach einem der vorhergehenden Ansprüche, wobei die hydrophobe kontinuierliche Phase Silikonöl, vorzugsweise Dimethicon, umfasst, das eine Viskosität von 1 bis 10.0000 mPas (Centistoke), vorzugsweise 5 bis 1000 mPas (Centistoke), mehr bevorzugt 25 bis 500 mPas (Centistoke) aufweist.

**7.** Verfahren nach einem der vorstehenden Ansprüche, wobei der Emulgator einen HLB-Wert von 1,5 bis 7 und mehr bevorzugt von 3 bis 6 aufweist.

**8.** Verfahren nach einem der vorstehenden Ansprüche, das zwischen Schritt (a) und (b) den folgenden zusätzlichen Schritt umfasst:

(a1) Einmassieren der Schutzzusammensetzung in die Haut für mindestens 10 Sekunden.

**9.** Verfahren nach einem der vorstehenden Ansprüche, das unmittelbar nach Schritt (b) den folgenden zusätzlichen Schritt umfasst:

(c) Belassen der Enthaarungszusammensetzung auf der Schutzzusammensetzung für einen Zeitraum von mindestens 1 Minute, vorzugsweise von 1 bis 10 Minuten, mehr bevorzugt von 3 bis 10 Minuten.

**10.** Verfahren nach Anspruch 9, das unmittelbar nach Schritt (c) den folgenden zusätzlichen Schritt umfasst:

(d) Entfernen sowohl der Schutzzusammensetzung als auch der Enthaarungszusammensetzung von der Haut, wahlweise durch Abschaben, Abwischen oder Abreiben.

**11.** Verfahren nach einem der vorstehenden Ansprüche, wobei das Thioglycolatsalz Kalium- oder Calciumthioglycolat oder Mischungen davon umfasst.

**12.** Enthaarungsset, umfassend:

(a) eine Schutzzusammensetzung, wobei die Schutzzusammensetzung einen Emulgator mit einem HLB-Wert von 1 bis 8 und eine Emulsion mit einer hydrophoben kontinuierlichen Phase und einer hydrophilen dispergierten Phase aufweist,
(b) eine Enthaarungszusammensetzung, die eine wirksame Menge eines Keratinreduktionsmittels umfasst., wobei das Keratinreduktionsmittel mindestens ein Thioglycolatsalz oder eine Thioglycolsäure umfasst.

**13.** Enthaarungsset nach Anspruch 12, wobei die Schutzzusammensetzung zu 0,1 Gew.-% bis 15 Gew.-%, vorzugsweise zu 0,5 Gew.-% bis 10 Gew.-%, mehr bevorzugt zu 1 Gew.-% bis 8 Gew.-% der Schutzzusammensetzung Wachs umfasst.

**14.** Enthaarungsset nach Anspruch 12 oder 13, das zusätzlich ein Werkzeug wie z. B. einen Schaber oder einen Spatel oder ein Wischtuch umfasst.

## Revendications

**1.** Procédé d'élimination de poils de la peau, comprenant les étapes consistant à:

(a) appliquer une composition protectrice sur une zone de peau sur laquelle poussent des poils indésirables, dans lequel la composition protectrice comprend un émulsifiant possédant un rapport hydro-lipophile allant de 1 à 8 et une émulsion possédant une phase continue hydrophobe et une phase dispersée hydrophile ;
(b) appliquer une composition dépilatoire sur la zone de peau sur laquelle la composition protectrice a été appliquée, la composition dépilatoire comprenant un agent réducteur de kératine, l'agent réducteur de kératine comprenant au moins un sel thioglycolate ou de l'acide thioglycolique.

**2.** Procédé selon la revendication 1, dans lequel l'émulsion comprend 10 % à 95 %, de préférence, 15 % à 95 %, plus préférablement 25 % à 75 % et plus préférablement encore 35 % à 65 % de phase continue hydrophobe en poids de la composition protectrice.

**3.** Procédé selon la revendication 1 ou 2, dans lequel la phase continue hydrophobe comprend une cire.

**4.** Procédé selon l'une quelconque des revendications 1 à 3, dans lequel la composition protectrice comprend 0,1 % à 15 %, de préférence 0,5 % à 10 %, plus préférablement 1 % à 8 % de cire en poids de la composition protectrice.

**5.** Procédé selon la revendication 3 ou 4, dans lequel la cire comprend une cire naturelle, une cire synthétique, une cire de silicone, ou leurs mélanges.

**6.** Procédé selon l'une quelconque des revendications précédentes, dans lequel la phase continue hydrophobe comprend une huile de silicone, de préférence de la diméthicone ayant une viscosité de 1 à 10 0000 (mPa.s (centistokes)), de préférence 5 à 1000 (mPa.s (centistokes)), plus préférablement 25 à 500 (mPa.s (centistokes)).

**7.** Procédé selon l'une quelconque des revendications précédentes, dans lequel l'émulsifiant a un rapport hydro-lipophile allant de 1,5 à 7 et plus préférablement de 3 à 6.

**8.** Procédé selon l'une quelconque des revendications précédentes, comprenant l'étape supplémentaire suivante entre l'étape (a) et l'étape (b) :

(a1) masser la composition protectrice dans la peau pendant au moins 10 secondes.

**9.** Procédé selon l'une quelconque des revendications précédentes, comprenant l'étape supplémentaire suivante immédiatement après l'étape (b) :

(c) laisser la composition dépilatoire en place sur la composition protectrice pendant une période d'au moins 1 minute, de préférence de 1 à 10 minutes, plus préférablement de 3 à 10 minutes.

**10.** Procédé selon la revendication 9, comprenant l'étape supplémentaire suivante immédiatement après l'étape (c) :

(d) éliminer à la fois la composition protectrice et la composition dépilatoire de la peau, facultativement en la raclant, l'essuyant ou la frottant.

**11.** Procédé selon l'une quelconque des revendications précédentes, dans lequel le sel thioglycolate comprend du thioglycolate de potassium ou de calcium, ou leurs mélanges.

**12.** Trousse dépilatoire comprenant :

(a) une composition protectrice, la composition protectrice comprenant un émulsifiant possédant un rapport hydro-lipophile allant de 1 à 8 et une émulsion possédant une phase continue hydrophobe et une phase dispersée hydrophile ;

(b) une composition dépilatoire comprenant une quantité efficace d'un agent réducteur de kératine, l'agent réducteur de kératine comprenant au moins un sel thioglycolate ou de l'acide thioglycolique.

**13.** Trousse dépilatoire selon la revendication 12, dans laquelle la composition protectrice comprend 0,1 % à 15 %, de préférence 0,5 % à 10 %, plus préférablement 1 % à 8 % de cire en poids de la composition protectrice.

**14.** Trousse dépilatoire selon les revendications 12 ou 13, comprenant en outre un outil, tel qu'un racloir ou une spatule, ou une lingette.

Fig.1.

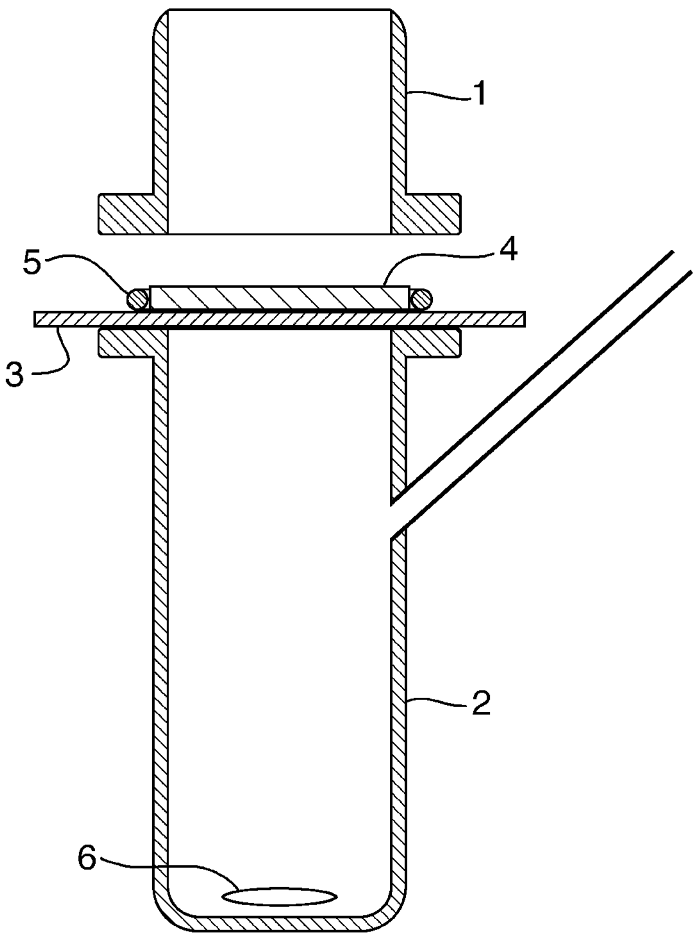

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4401663 A
- US 4424205 A
- US 20040219118 A


### Non-patent literature cited in the description


- The HLB System, A Time-Saving Glide to Emulsifier Selection. ICI Americas Inc, 1984
- McCutcheon's Detergents and Emulsifiers. 1986, 317-324
- The Encyclopaedia of Polymers and Thickeners for Cosmetics. Department of Polymer Science